# EUROPEAN PATENT APPLICATION

(11) **EP 4 692 270 A1**
(43) Date of publication of application: **11.02.2026**
(21) Application number: 24779974.5
(22) Date of filing: 22.03.2024
(51) Int. Cl.: C09K 3/00, A61K 9/06, A61K 47/36, A61K 47/38, C12M 3/00

(54) **GELLING AGENT, LIQUID COMPOSITION, AND HYDROGEL**

(30) Priority: 24.03.2023 JP 2023048520
(71) Applicant: INSTITUTE OF SCIENCE TOKYO, Tokyo 152-8550 (JP); DKS Co. Ltd., Kyoto-shi, Kyoto 600-8873 (JP)
(72) Inventor: SERIZAWA Takeshi, Tokyo 152-8550 (JP); SAWADA Toshiki, Tokyo 152-8550 (JP); HAYAKAWA Natsuki, Kyoto-shi, Kyoto 600-8873 (JP); NISHIURA Masahito, Kyoto-shi, Kyoto 600-8873 (JP)
(74) Representative: Hoffmann Eitle
(86) International application number: PCT/JP2024/011311
(87) International publication number: WO 2024/203856

(57) **Abstract**

Provided are a novel gelling agent, a gellable novel liquid composition using the same, and a hydrogel. A gelling agent according to an embodiment is a gelling agent for gelling a polysaccharide such as hyaluronic acid, and includes a cellulose oligomer having an average degree of polymerization of 5 or more and 20 or less. A gellable liquid composition according to an embodiment includes a cellulose oligomer having an average degree of polymerization of 5 or more and 20 or less, a polysaccharide, and water.

## Description

### Technical Field

The present invention relates to a gelling agent, a gellable liquid composition, and a hydrogel formed by the gelation of the liquid composition.

### Background Art

Gels formed from a composition containing cellulose or a derivative thereof and a polysaccharide, such as hyaluronic acid, are known. For example, PTL 1 discloses that an aqueous solution with a pH of 3.5 or less containing hyaluronic acid and carboxymethylcellulose is frozen and then thawed, thereby producing a co-crosslinked gel composition composed of hyaluronic acid and carboxymethylcellulose.

Meanwhile, PTL 2 discloses use of a cellulose oligomer having an average degree of polymerization of 5 to 20 as a medium additive to be added to a medium for culturing cells. A cellulose oligomer is an oligosaccharide having a structure in which glucose is linked by a β-1,4-glycosidic bond. In PTL 2, the cellulose oligomer is added in order to suspend cells in the medium, and it is nowhere disclosed that gelation is caused by combining a cellulose oligomer with a polysaccharide.

### Citation List

### Patent Literature

PTL 1: JP2003-019194A
PTL 2: JP2021-069339A

### Summary of Invention

### Technical Problem

In the course of investigating new applications of the cellulose oligomer, the present inventors have found that a cellulose oligomer forms a hydrogel through interaction with a polysaccharide such as hyaluronic acid.

Embodiments of the invention are based on the above findings, and an object thereof is to provide a novel gelling agent capable of gelling a polysaccharide, a gellable liquid composition containing a polysaccharide, and a hydrogel formed by the gelation of the liquid composition.

### Solution to Problem

The invention includes the following embodiments.
[1] A gelling agent for gelling a polysaccharide, the gelling agent including a cellulose oligomer having an average degree of polymerization of 5 or more and 20 or less.
[2] A gellable liquid composition including a cellulose oligomer having an average degree of polymerization of 5 or more and 20 or less, a polysaccharide, and water.
[3] The liquid composition according to [2] gellable by neutralization with an acid, in which the liquid composition is an alkaline aqueous solution containing the cellulose oligomer and the polysaccharide.
[4] The liquid composition according to [2], obtained by neutralizing an alkaline aqueous solution containing the cellulose oligomer and the polysaccharide with an acid to precipitate the cellulose oligomer.
[5] The liquid composition according to [2] or [3], in which the polysaccharide is a non-cellulosic polysaccharide.
[6] The liquid composition according to [2] or [3], in which the polysaccharide is at least one selected from the group consisting of a glycosaminoglycan and a water-soluble polysaccharide that is composed only of glucose and/or a glucose derivative polymerized through an α-1,4-glycosidic bond and an α-1,6-glycosidic bond.
[7] The liquid composition according to any one of [2] to [6], including 0.5 to 100 parts by mass of the cellulose oligomer per 100 parts by mass of the polysaccharide.
[8] A hydrogel formed by the gelation of the liquid composition according to any one of [2] to [7].

### Advantageous Effects of Invention

According to embodiments of the invention, it is possible to provide a novel gelling agent capable of gelling a polysaccharide, a gellable liquid composition containing a polysaccharide, and a hydrogel formed by the gelation of the liquid composition.

### Brief Description of Drawings

[FIG. 1] A conceptual diagram for explaining the presumed mechanism of gelation according to one embodiment
[FIG. 2] Photographs showing the conditions 24 hours after inversion in Examples 1 to 3 and Comparative Examples 1 to 4

### Description of Embodiments

### [Gelling Agent]

A gelling agent according to an embodiment is a gelling agent for gelling a polysaccharide and includes a cellulose oligomer having an average degree of polymerization of 5 to 20.

A cellulose oligomer is an oligosaccharide having a structure in which glucose is linked by a β-1,4-glycosidic bond, and is also referred to as "cellooligosaccharide". The cellulose oligomer may have no substituent, or may have a substituent at the anomeric position of the reducing end.

In this embodiment, as the cellulose oligomer, one in which the average degree of polymerization (DP) (the average number of glucose units present in one molecule) is 5 to 20 is used. From the viewpoint that a more uniform gel can be formed, the average degree of polymerization (DP) is preferably 5 to 18, more preferably 6 to 15, still more preferably 6 to 10, and yet more preferably 7 to 9. The average degree of polymerization of a cellulose oligomer is the weighted average value of the degree of polymerization according to the mass ratio of the cellulose oligomer. A cellulose oligomer is usually a mixture of compounds having different degrees of polymerization, and may include, for example, those having a degree of polymerization of 4 to 20, those having a degree of polymerization of 5 to 18, or those having a degree of polymerization of 5 to 13.

As the cellulose oligomer, it is preferable to use a compound represented by the following general formula (1).

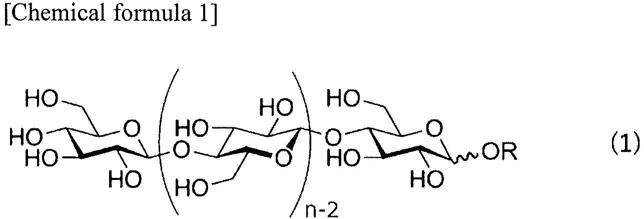

In formula (1), R represents a hydrogen atom or a substituent introduced in place of a hydrogen atom. Incidentally, the wavy line in the bond between the carbon at position 1 of the reducing end (anomeric carbon) and the OR group indicates that the stereochemical configuration of the OR group is α-form, β-form, or a mixture of α-form and β-form. The stereochemical configuration of the OR group is preferably β-form.

In formula (1), n is an average degree of polymerization and represents a number of 5 to 20. n is preferably 5 to 18, more preferably 6 to 15, still more preferably 6 to 10, and yet more preferably 7 to 9.

The method for synthesizing a cellulose oligomer is not particularly limited. For example, a method in which α-glucose-1-phosphate (hereinafter sometimes referred to as αG1P) and at least one primer selected from the group consisting of glucose, cellobiose, and a derivative thereof are allowed to react with cellodextrin phosphorylase (hereinafter sometimes referred to as CDP) can be mentioned. This reaction is a synthesis method utilizing the reverse reaction of CDP, in which αG1P is used as a glucose donor, while at least one selected from the group consisting of glucose, cellobiose, and a derivative thereof is used as a primer (i.e., a glucose acceptor), and they are allowed to react with CDP, whereby αG1P is sequentially polymerized as a monomer onto the primer. Incidentally, when the hydroxyl group at the anomeric position in the primer has its hydrogen atom replaced with a substituent, cellulose oligomers having various substituents can be synthesized.

As the cellulose oligomer, one synthesized using CDP as described above may be used as it is, or one dissolved in an alkaline aqueous solution may also be used. Alternatively, it is also possible to use a self-assembled cellulose oligomer.

A self-assembled cellulose oligomer can be prepared as follows. That is, a cellulose oligomer is dissolved in an alkaline aqueous solution to prepare an aqueous solution, and the obtained aqueous solution is neutralized with an acid. As a result, the cellulose oligomer becomes insoluble and precipitates, which is then incubated with stirring, shaking, etc., or while being left to stand, whereby the cellulose oligomer self-assembles. That is, molecules of the cellulose oligomer are aligned, forming a cellulose oligomer assembly having a cellulose II type crystal structure. In one embodiment, the cellulose oligomer may be a cellulose oligomer assembly having a cellulose II type crystal structure, which contains a compound represented by formula (1) as a constituent component.

The cellulose oligomer assembly may have a sheet-like structure (cellulose nanosheet). Here, the sheet-like structure is a concept that encompasses an elongated, ribbon-like structure (cellulose nanoribbon).

In one embodiment, the gelling agent may be a water dispersion of the cellulose oligomer. That is, the gelling agent may be a dispersion liquid composed of the cellulose oligomer assembly dispersed in water. Alternatively, in one embodiment, the gelling agent may be an aqueous solution of the cellulose oligomer dissolved in an alkaline aqueous solution. Alternatively, in one embodiment, the gelling agent may be in the form of a powder obtained by drying a water dispersion or aqueous solution of the cellulose oligomer through freezedrying or the like.

In the case where the gelling agent is the water dispersion described above, the content (concentration) of the cellulose oligomer is not particularly limited, and is, for example, preferably 0.10 to 15% (w/v), more preferably 0.12 to 5.0% (w/v), and still more preferably 0.12 to 1.0% (w/v).

In the case where the gelling agent is the aqueous solution described above, the content (concentration) of the cellulose oligomer is not particularly limited, and is, for example, preferably 0.12 to 1.3% (w/v), and more preferably 0.14 to 0.70% (w/v).

As used herein, "% (w/v)" is a mass/volume percentage concentration, which is the mass (g) of the target substance per 100 mL of volume.

The gelling agent may contain, together with the cellulose oligomer and water as a dispersion medium or solvent, various additives such as alkali agents, buffer agents, and inorganic salts, for example.

As alkaline agents, for example, sodium hydroxide, potassium hydroxide, lithium hydroxide, and the like can be mentioned.

As buffering agents, for example, buffering agents constituting phosphate buffered saline (PBS), 2-morpholinoethanesulfonic acid (MES) buffer, trishydroxymethylaminomethane (Tris) buffer, 3-morpholinopropanesulfonic acid (MOPS) buffer, 4-(2-hydroxyethyl)-1-piperazineethanesulfonic acid (HEPES) buffer, and the like can be mentioned.

As inorganic salts, for example, sodium chloride, potassium chloride, lithium chloride, and the like can be mentioned.

The gelling agent is used to gel a polysaccharide. As the polysaccharide, a non-cellulosic polysaccharide is preferably used. More preferably, as polysaccharides, (A) glycosaminoglycans and (B) water-soluble polysaccharides composed only of glucose and/or a glucose derivative polymerized through an α-1,4-glycosidic bond and an α-1,6-glycosidic bond can be mentioned. Any one of them may be used alone, and it is also possible to use two or more kinds together. Many of them are constituent polysaccharides of the extracellular matrix and are preferable for use in this embodiment.

A glycosaminoglycan of the above (A) is a polysaccharide containing amino sugars having a structure in which the hydroxy group of a sugar is substituted with an amino group. As glycosaminoglycans, for example, hyaluronic acid, chondroitin sulfate, heparin, keratan sulfate, heparan sulfate, and the like can be mentioned.

A water-soluble polysaccharide of the above (B) is a polysaccharide having a structure in which glucose and/or a glucose derivative is polymerized through an α-1,4-glycosidic bond and an α-1,6-glycosidic bond, and, for example, dextran and derivatives thereof (e.g., carboxymethyl dextran (CM-Dex), etc.), pullulan, and the like can be mentioned.

Incidentally, as used herein, in the case where the above polysaccharides have an anionic group, the concept thereof also encompasses their salts, such as sodium salts.

In one embodiment, the polysaccharide is preferably at least one selected from the group consisting of hyaluronic acid, chondroitin sulfate, heparin, carboxymethyl dextran, and pullulan.

### [Liquid Composition]

A liquid composition according to an embodiment contains the gelling agent described above, and thus is a gellable liquid composition containing a cellulose oligomer having an average degree of polymerization of 5 or more and 20 or less, a polysaccharide, and water.

In the liquid composition, the cellulose oligomer and the polysaccharide are as described above, and descriptions thereof will be omitted.

In the liquid composition, the content of the cellulose oligomer is preferably 0.5 to 100 parts by mass per 100 parts by mass of the polysaccharide. Because the cellulose oligomer is used as a gelling agent for gelling an aqueous solution of the polysaccharide, the content of the cellulose oligomer is preferably equal to or lower than the content of the polysaccharide. The content of the cellulose oligomer is preferably 1 to 80 parts by mass, more preferably 2 to 70 parts by mass, still more preferably 3 to 60 parts by mass, and yet more preferably 4 to 50 parts by mass, per 100 parts by mass of the polysaccharide.

In one embodiment, in the case where hyaluronic acid is used as the polysaccharide, the content of the cellulose oligomer is preferably 5 to 100 parts by mass, more preferably 8 to 80 parts by mass, still more preferably 10 to 60 parts by mass, and yet more preferably 15 to 40 parts by mass, per 100 parts by mass of hyaluronic acid.

In the liquid composition, the content (concentration) of the cellulose oligomer is not particularly limited, and is, for example, preferably 0. 10 to 1.0% (w/v), more preferably 0.12 to 0.50% (w/v), still more preferably 0.15 to 0.40% (w/v), yet more preferably 0.18 to 0.35% (w/v), and still yet more preferably 0.20 to 0.30% (w/v).

In the liquid composition, the content (concentration) of the polysaccharide is not particularly limited, and may be, for example, 0.30 to 10.0% (w/v), 0.50 to 8.0% (w/v), 0.60 to 6.0% (w/v), or 0.80 to 5.0% (w/v). In one embodiment, in the case where hyaluronic acid is used as the polysaccharide, the content (concentration) of hyaluronic acid in the liquid composition is not particularly limited, but is preferably 0.50 to 1.5% (w/v), more preferably 0.60 to 1.3% (w/v), and still more preferably 0.80 to 1.2% (w/v).

In the liquid composition, the cellulose oligomer may be contained in the form of being dissolved in an alkaline aqueous solution, or may be contained in the form of being dispersed in water as a cellulose oligomer assembly.

For example, in one embodiment, the liquid composition may be an alkaline aqueous solution containing a cellulose oligomer and a polysaccharide. In that case, the liquid composition can be gelled by neutralization with an acid.

In addition, in one embodiment, the liquid composition may contain a cellulose oligomer assembly dispersed in water and a polysaccharide dissolved in water. In that case, the gellable liquid composition can be obtained by neutralizing an alkaline aqueous solution containing a cellulose oligomer and a polysaccharide with an acid to precipitate the cellulose oligomer.

The liquid composition may contain, together with the cellulose oligomer, the polysaccharide, and water, various additives such as alkaline agents, buffering agents, inorganic salts, organic low-molecular-weight compounds, and proteins, for example. Alkaline agents, buffering agents, and inorganic salts are as described above, and descriptions thereof will be omitted.

### [Hydrogel]

The liquid composition can be gelled to form a hydrogel. Therefore, a hydrogel according to an embodiment is formed by the gelation of the liquid composition described above. Thus, the hydrogel contains the cellulose oligomer, the polysaccharide, and water, and may further contain various additives contained in the liquid composition. Incidentally, the contents of the cellulose oligomer and the polysaccharide in the hydrogel and the ratio between the two are as described above for the liquid composition, and descriptions thereof will be omitted.

Here, a hydrogel refers to a dispersion system using water as the dispersion medium, which has lost fluidity and solidified. In detail, in the gelation evaluation test described below, the vial is inverted, and if the dispersion system is all retained at the top of the vial without flowing down to the bottom of the vial at all after a lapse of 10 minutes, it is judged that gelation has occurred (i.e., a hydrogel has been formed).

The liquid composition has temperature responsiveness with respect to the gelation ability. That is, the liquid composition gels upon heating (or can be gelled more quickly than under low-temperature conditions), and can maintain its gel state even if subsequently cooled.

The hydrogel is formed as a result of the complexation of a cellulose oligomer and a polysaccharide due to the interaction between the two. In detail, it is believed that in the hydrogel, the cellulose oligomer has self-assembled and is contained as a cellulose oligomer assembly, and the cellulose oligomer assembly undergoes aggregation to function as a crosslinking agent that connects the molecular chains of the polysaccharide (physical crosslinking).

As a method for forming a hydrogel, for example, it is possible that a cellulose oligomer is self-assembled to form a water dispersion of a cellulose oligomer assembly, and then the water dispersion is mixed with a polysaccharide or an aqueous solution thereof. However, it is preferable that an alkaline aqueous solution containing a cellulose oligomer and a polysaccharide is neutralized with an acid to precipitate the cellulose oligomer. As a result of this, a more uniform hydrogel can be formed.

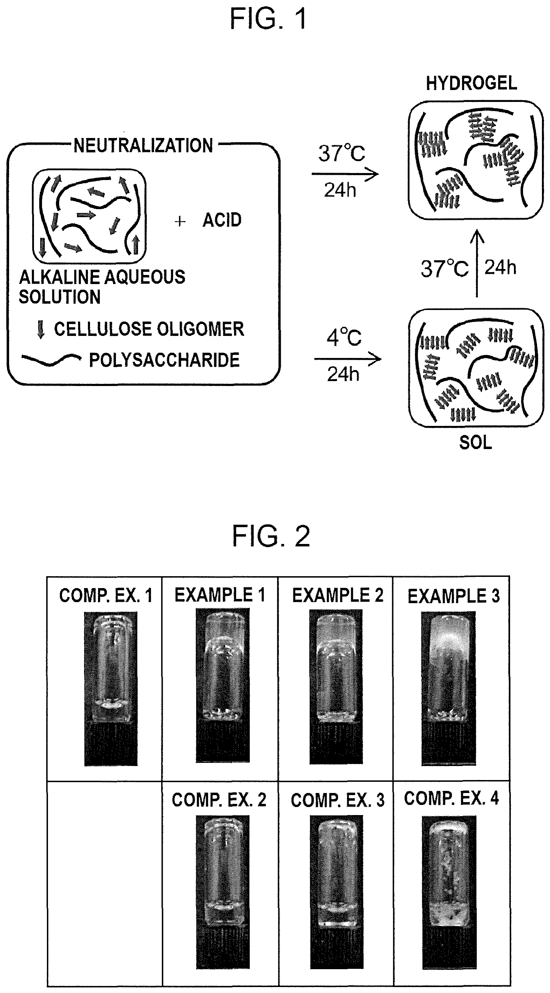

FIG. 1 is a conceptual diagram showing the presumed mechanism of gelation according to one embodiment. Here, a case where a hydrogel is formed by neutralizing an alkaline aqueous solution will be described. An alkaline aqueous solution having dissolved therein a cellulose oligomer and a polysaccharide is neutralized by adding an acid thereto (adjusted to neutral pH). At that time, the acid may have added thereto a buffer. In the case where the aqueous solution after neutralization is placed in a high-temperature environment (e.g., 37°C) for a predetermined period of time (e.g., 24 hours), the fluidity of the aqueous solution is lost, and a hydrogel is formed. In contrast, in the case where the aqueous solution after neutralization is placed in a low-temperature environment (e.g., 4°C) for a predetermined period of time (e.g., 24 hours), the aqueous solution maintains its fluidity, and a sol is formed. Then, when the sol is placed in a high-temperature environment (e.g., 37°C) for a predetermined period of time (e.g., 24 hours), its fluidity is lost, and a hydrogel is formed.

The mechanism of such gelation is presumed to be as follows. As a result of the neutralization of the alkaline aqueous solution, the cellulose oligomer becomes insoluble and precipitates, and then self-assembles, forming a cellulose oligomer assembly. At that time, it is believed that because a polysaccharide is present in the aqueous solution, as shown in FIG. 1, an interaction occurs between the self-assembly of the cellulose oligomer and the molecular chains of the polysaccharide, while in a high-temperature environment, the cellulose oligomer assembly undergoes aggregation, and such an aggregate serves as a crosslinking agent that connects the molecular chains of the polysaccharide. Accordingly, the molecular chains of the polysaccharide and the aggregates form a hydrogel having a three-dimensional network.

Meanwhile, it is believed that in a low-temperature environment, although an interaction occurs between the cellulose oligomer assembly and the molecular chains of the polysaccharide, the cellulose oligomer assembly does not undergo aggregation (or the aggregation speed is so slow that aggregation sufficient to cause loss of fluidity does not occur within a predetermined period of time), resulting in the formation of a sol having fluidity. It is believed that when the sol is placed in a high-temperature environment, the aggregation of the cellulose oligomer assembly is promoted, and a crosslinked structure that connects the molecular chains of the polysaccharide is formed, forming a hydrogel having a three-dimensional network.

The temperature for gelling the liquid composition is not particularly limited, and may be, for example, 15°C or more, 20°C or more, or 25°C or more. The upper limit of the gelling temperature is not particularly limited either, and may be, for example, 60°C or less, or 50°C or less. The time for gelling the liquid composition is not particularly limited, and may be 10 hours or more, 15 hours or more, or 18 hours or more.

### [Application]

The application of the hydrogel according to the embodiment is not particularly limited, and examples thereof include a scaffold for cell culture. Examples also include a drug delivery system application, in which a drug or useful protein is placed in the hydrogel to control its distribution in the body.

### Examples

Hereinafter, the invention will be further described with reference to examples, but is not limited thereto.

### [Synthesis of Cellulose Oligomer]

In accordance with the method described in T. Serizawa et al., Polym. J., 2016, 48, 539 to 544 (using D-glucose as a primer), a cellulose oligomer (glucose-derived) having an average degree of polymerization of 9 (n = 9) represented by the following formula was synthesized. In addition, in accordance with the method described in T. Serizawa et al., Langmuir, 2017, 33, 13415 to 13422 (using cellobiose as a primer), a cellulose oligomer (cellobiose-derived) having an average degree of polymerization of 7 (n = 7) represented by the following formula was synthesized.

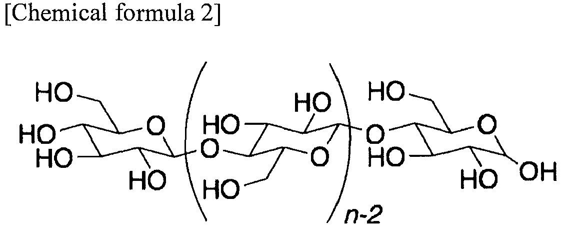

In detail, 200 mmol/L of αG1P, 50 mmol/L of D-glucose or cellobiose, and 0.2 U/mL of cellodextrin phosphorylase (CDP) were mixed in 500 mmol/L of 2-[4-(2-hydroxyethyl)-1-piperazinyl]ethanesulfonic acid (HEPES) buffer (pH 7.5), and incubated at 60°C for 3 days. The reaction mixture containing the product was centrifuged (15,000 rpm, 10 minutes or more, 4°C), the supernatant was removed, and then ultrapure water was added to redisperse the product, followed by centrifugation (same conditions); this operation was repeated for purification until the supernatant replacement rate reached 99.999% or more, thereby giving a cellulose oligomer.

The average degree of polymerization of the product was measured using a proton nuclear magnetic resonance (NMR) apparatus. The sample was prepared by dissolving 12 mg or more of the lyophilized product in 600 µL of 4 mass% sodium deuteroxide in deuterium oxide. As the NMR apparatus, ADVANCE III HD500 (Bruker Biospin, magnetic field strength: 500 MHz, number of accumulations: 16) was used. The average degree of polymerization was calculated based on the integrals of the protons at the anomeric position of the reducing end of the cellulose oligomer (δ ≈ 5.1, 4.5) and other anomeric positions (δ ≈ 4.3).

### [Polysaccharide]

The polysaccharides used in the examples are as follows.
- Hyaluronic acid: manufactured by Nacalai Tesque
- CM-Dex: manufactured by Tokyo Chemical Industry Co., Ltd.
- Chondroitin sulfate C sodium: manufactured by Nacalai Tesque
- Sodium heparin: manufactured by Nacalai Tesque

### [Test Example 1: Examples 1 to 3, Comparative Examples 1 to 4]

A 1-mL mighty vial was used as a container. A 1 mol/L aqueous NaOH solution having dissolved therein a cellulose oligomer and hyaluronic acid was neutralized with a 1 mol/L aqueous HCl solution to prepare 300 µL of an aqueous solution. At that time, the aqueous HCl solution had added thereto a phosphate buffer component so that the aqueous solution after neutralization was phosphate buffered saline (PBS, [NaCl] 137 mmol/L). In addition, the concentrations of the cellulose oligomer and hyaluronic acid in the aqueous solution after neutralization were set to be as shown in Table 1 below. In addition, the types of cellulose oligomers are as shown in Table 1. The obtained aqueous solution was left to stand in a 25°C atmosphere for 24 hours, followed by gelation evaluation.

In the gelation evaluation, the vial after standing was inverted, and if, 10 minutes after inversion, the aqueous solution (dispersion system) was all retained at the top of the vial without flowing down to the bottom of the vial at all, gelation was judged to have occurred. In Table 1, those that gelled are indicated as "gel", and those that did not are indicated as "sol".

**[Table 1]**

| | Comp. Ex. 1 | Ex. 1 | Ex. 2 | Comp. Ex. 2 | Comp. Ex. 3 | Ex. 3 | Comp. Ex. 4 |
|---|---|---|---|---|---|---|---|
| Cellulose Oligomer Type | - | Cellobiose-derived | | | | Glucose-derived | |
| Cellulose Oligomer Concentration [% (w/v)] | 0 | 0.20 | 0.30 | 0.20 | 0.30 | 0.20 | 0.20 |
| Hyaluronic Acid Concentration [% (w/v) | 1.0 | 1.0 | 1.0 | 0 | 0 | 1.0 | 0 |
| Gelation Evaluation | Sol | Gel | Gel | Sol | Sol | Gel | Sol |

The results are as shown in Table 1. In Comparative Example 1 where no cellulose oligomer was incorporated, gelation did not occur. Also in Comparative Examples 2 and 3 and Comparative Example 4 where no hyaluronic acid was incorporated, gelation did not occur. In contrast, in each of Examples 1 to 3 where a cellulose oligomer and hyaluronic acid were used together, gelation occurred, and a hydrogel resulting from the complexation of a cellulose oligomer and hyaluronic acid due to the interaction between the two was obtained.

FIG. 2 is photographs showing the conditions 24 hours after inversion. In each of Examples 1 to 3, even after a lapse of 24 hours, the solution did not flow down, and the gel state was maintained. When the types of cellulose oligomers were compared, in Examples 1 and 2 where a cellobiose-derived cellulose oligomer was used, the formed gels were more transparent and uniform than in Example 3 where a glucose-derived cellulose oligomer was used.

### [Test Example 2: Comparative Examples 5 and 6]

In a 1-mL mighty vial, 300 µL of an aqueous solution having dissolved therein sodium carboxymethylcellulose (manufactured by Nacalai Tesque) and hyaluronic acid was prepared. At that time, the concentration of sodium carboxymethylcellulose in the aqueous solution was set to 0.20% (w/v) in Comparative Example 5 and 0.30% (w/v) in Comparative Example 6. In addition, the concentration of hyaluronic acid was set to 1.0% (w/v) in both Comparative Examples 5 and 6. The obtained aqueous solutions were left to stand in a 25°C atmosphere for 24 hours, followed by gelation evaluation in the same manner as in Test Example 1.

As a result, in both Comparative Examples 5 and 6 where sodium carboxymethylcellulose was used in place of a cellulose oligomer, 10 minutes after the vial after standing was inverted, it was seen that the aqueous solution was flowing down, and gelation did not occur.

### [Test Example 3: Examples 4 to 6]

In the same manner as in Example 1 except that the concentration of hyaluronic acid in the aqueous solution after neutralization was set to 0.40% (w/v) in Example 4, 0.60% (w/v) in Example 5, and 0.80% (w/v) in Example 6, aqueous solutions after neutralization were obtained and left to stand in a 25°C atmosphere for 24 hours, followed by gelation evaluation. As a result, in each of Examples 4 to 6, 10 minutes after the vial after standing was inverted, the aqueous solution did not flow down, and gelation had occurred. In addition, in each of Examples 4 to 6, even after a lapse of 24 hours from inversion, the solution did not flow down, and the gel state was maintained.

### [Test Example 4: Examples 7 to 9, Comparative Examples 7 to 12: Standing Temperature Dependence]

In a 1-mL mighty vial, a 1 mol/L aqueous NaOH solution having dissolved therein a cellulose oligomer and hyaluronic acid was neutralized with a 1 mol/L aqueous HCl solution having added thereto a phosphate buffer component, thereby preparing 300 µL of an aqueous solution such that the aqueous solution after neutralization was phosphate buffered saline (PBS, [NaCl] 137 mmol/L). At that time, the concentration of the cellulose oligomer (cellobiose-derived) and the concentration of hyaluronic acid in the aqueous solution after neutralization were set to 0.20% (w/v) and 1.0% (w/v), respectively. The obtained aqueous solutions were left to stand in a 4°C atmosphere in Example 7, a 25°C atmosphere in Example 8, and a 37°C atmosphere in Example 9, respectively, for 24 hours, followed by gelation evaluation in the same manner as in Test Example 1.

In Comparative Examples 7 to 9, aqueous solutions were prepared in the same manner as in Examples 7 to 9 except that no cellulose oligomer was added, and the obtained aqueous solutions were left to stand in a 4°C atmosphere in Comparative Example 7, a 25°C atmosphere in Comparative Example 8, and a 37°C atmosphere in Comparative Example 9, respectively, for 24 hours, followed by gelation evaluation in the same manner as in Test Example 1.

In Comparative Examples 10 to 12, aqueous solutions were prepared in the same manner as in Examples 7 to 9 except that no hyaluronic acid was added, and the obtained aqueous solutions were left to stand in a 4°C atmosphere in Comparative Example 10, a 25°C atmosphere in Comparative Example 11, and a 37°C atmosphere in Comparative Example 12, respectively, for 24 hours, followed by gelation evaluation in the same manner as in Test Example 1.

The results are as shown in Table 2 below. In Comparative Examples 7 to 9 where no cellulose oligomer was added, and only hyaluronic acid was incorporated, regardless of whether the solution was left to stand in a 4°C, 25°C, or 37°C atmosphere, gelation did not occur. Similarly, in Comparative Examples 10 to 12 where no hyaluronic acid was added, and only cellulose oligomer was incorporated, regardless of whether the solution was placed in a 4°C, 25°C, or 37°C atmosphere, gelation did not occur. In contrast, in the case where a cellulose oligomer and hyaluronic acid were incorporated, although gelation did not occur in Example 7 where the solution was left to stand at 4°C, in Examples 8 and 9 where the solutions were left to stand at 25°C and 37°C, as well as after a lapse of 10 minutes from inversion, the gel state was maintained also after a lapse of 24 hours from inversion. As a result of this, it was found that gelation can be controlled by the standing temperature. In addition, there was a tendency that the cloudiness of the gel increased with an increase in the standing temperature. This suggested the possibility that the aggregation of a cellulose oligomer assembly may contribute to gelation.

The vial containing the sol of Example 7 that had not gelled was placed in an upright position and then left to stand in a 37°C atmosphere for 24 hours, followed by gelation evaluation in the same manner. As a result, the sol had gelled, and the gel state was maintained even after a lapse of 24 hours from inversion. Further, the vial in the gel state was placed in an upright position, left to stand in a 4°C atmosphere for 24 hours, and then inverted. As a result, the gel state was maintained after a lapse of 24 hours from inversion. It was found that when a product in liquid state (sol) obtained through standing at 4°C is left to stand at 37°C in this way, the sol becomes a gel. In addition, after the sol became a gel in this way, even when the temperature was returned to 4°C, it did not return to the liquid state. That is, it was found that a gel once formed is stable against temperature changes. This suggested that a cellulose oligomer assembly that has once undergone aggregation does not disperse even when the temperature is lowered.

**[Table 2]**

| | Ex. 7 | Ex. 8 | Ex. 9 | Comp. Ex. 7 | Comp. Ex. 8 | Comp. Ex. 9 | Comp. Ex. 10 | Comp. Ex. 11 | Comp. Ex. 12 |
|---|---|---|---|---|---|---|---|---|---|
| Cellulose Oligomer Concentration [% (w/v)] | 0.20 | 0.20 | 0.20 | 0 | 0 | 0 | 0.20 | 0.20 | 0.20 |
| Hyaluronic Acid Concentration [% (w/v)] | 1.0 | 1.0 | 1.0 | 1.0 | 1.0 | 1.0 | 0 | 0 | 0 |
| Stading Temperature [°C] | 4 | 25 | 37 | 4 | 25 | 37 | 4 | 25 | 37 |
| Gelation Evaluation | Sol | Gel | Gel | Sol | Sol | Sol | Sol | Sol | Sol |
| After Further Stading at 37°C | Gel | | | | | | | | |
| After Further Stading at 4°C | Gel | | | | | | | | |

### [Test Example 5: Standing Time Dependence]

In the same manner as in Example 1, an aqueous solution after neutralization (hyaluronic acid concentration: 1.0% (w/v), cellulose oligomer (cellobiose-derived) concentration: 0.20% (w/v), NaCl concentration: 137 mmol/L (PBS condition)) was prepared. The obtained aqueous solution after neutralization was left to stand in a 25°C atmosphere for each of the standing times shown in Table 3 below, followed by gelation evaluation in the same manner as in Test Example 1.

The results are as shown in Table 3 below, and it was found that the formulation of Example 1 gelled when left to stand for 18 hours or more. It is believed that the self-assembly of a cellulose oligomer takes a shorter period of time. Thus, this supported the possibility that the aggregation of a cellulose oligomer assembly may contribute to gelation.

**[Table 3]**

| Stading Time | 10 minutes | 1 hour | 6 hours | 18 hours | 24 hours |
|---|---|---|---|---|---|
| Gelation Evaluation | Sol | Sol | Sol | Gel | Gel |

### [Test Example 6: Hydrogel Stability Evaluation]

In the same manner as in Example 1, an aqueous solution after neutralization (hyaluronic acid concentration: 1.0% (w/v), cellulose oligomer (cellobiose-derived) concentration: 0.20% (w/v), NaCl concentration: 137 mmol/L (PBS condition)) was prepared, and the obtained aqueous solution after neutralization was left to stand in a 25°C atmosphere for 24 hours to form a hydrogel of Example 10. In addition, in the same manner as in Example 10 except that the cellulose oligomer concentration was set to 1.0% (w/v), a hydrogel of Example 11 was formed. Next, 600 µL of ultrapure water was poured into each of the vials of Examples 10 and 11 to form a layer of ultrapure water on the hydrogel, and the vials were then left to stand in a 25°C atmosphere. The conditions of the hydrogels were observed from immediately after the start of standing to 60 days later.

As a result, in both Examples 10 and 11, the hydrogel maintained the gel state for at least 2 months after the addition of ultrapure water. In Example 11 where the cellulose oligomer concentration was 1.0% (w/v), after about a lapse of 6 days from the start of standing, it was observed that the hydrogel was floating up in the vial. This suggested that in Example 11, the internal salts were leaching out due to osmotic pressure. However, the shape of the hydrogel itself was kept unchanged. This suggested the possibility that the hydrogels of Examples 10 and 11 were stable in ultrapure water, and a network structure strong enough to not collapse upon immersion in ultrapure water had been constructed.

### [Test Example 7: Example 12: Low-Temperature, Long-Term Standing]

In the same manner as in Example 7, an aqueous solution after neutralization (hyaluronic acid concentration: 1.0% (w/v), cellulose oligomer (cellobiose-derived) concentration: 0.20% (w/v), NaCl concentration: 137 mmol/L (PBS condition)) was prepared, and the obtained aqueous solution after neutralization was left to stand in a 4°C atmosphere for 24 hours. When the vial was inverted, the aqueous solution flowed down, and gelation had not occurred. After the vial was left to stand in the inverted position at 4°C for 24 hours, the aqueous solution accumulated in the mouth of the vial was dropped off, the vial was returned to an upright position again, and then left to stand in a 4°C atmosphere. Then, after a total of two weeks of standing following neutralization, it was observed that the solution was cloudy. Next, the vial was inverted to evaluate gelation; as a result, after 10 minutes, the solution did not flow down, and gelation had occurred. The vial was further left to stand in the inverted position at 25°C for 24 hours; as a result, the gel state was still maintained even after a lapse of 24 hours. From this, it was found that even in the case of standing at 4°C, gelation occurs when the standing time is increased. This is believed to be because in a low-temperature environment of 4°C, the aggregation of a cellulose oligomer assembly takes a long period of time.

### [Test Example 8: Examples 13 to 17 and Comparative Examples 13 to 15]

Aqueous solutions after neutralization were prepared in the same manner as in Test Example 1 except that CM-Dex, chondroitin sulfate C sodium, and heparin sodium were used at a concentration of 5.0% (w/v) in place of hyaluronic acid at 1.0% (w/v) according to Table 4 below, and the concentration of the cellulose oligomer (cellobiose-derived) was set as shown in Table 4. The obtained aqueous solutions were left to stand in a 25°C atmosphere for 24 hours, followed by gelation evaluation.

The results are as shown in Table 4 below. Regardless of whether CM-Dex, chondroitin sulfate C sodium, or heparin sodium was used as a polysaccharide, in Comparative Examples 13 to 15 where no cellulose oligomer was incorporated, gelation did not occur. In contrast, in Examples 13 to 17 where a cellulose oligomer was incorporated together with these polysaccharides, the aqueous solutions did not flow down 10 minutes after inversion, and gelation had occurred.

**[Table 4]**

| | Comp. Ex. 13 | Ex. 13 | Ex. 14 | Comp. Ex. 14 | Ex. 15 | Ex. 10 | Comp. Ex. 15 | Ex. 17 |
|---|---|---|---|---|---|---|---|---|
| Cellulose Oligomer Concentration [% (w/v)] | 0 | 0.20 | 0.30 | 0 | 0.20 | 0.30 | 0 | 0.30 |
| Polysaccharide | CM-Dex 5.0% (w/v) | | | Chondroitin sulfate C sodium 5.0% (w/v) | | | Heparin sodium 5.0% (w/v) | |
| Gelation Evaluation | Sol | Gel | Gel | Sol | Gel | Gel | Sol | Gel |

Incidentally, with respect to the various numerical ranges described herein, the upper and lower limits thereof can be arbitrarily combined, and all such combinations are incorporated herein as preferred numerical ranges. In addition, the description of a numerical range "X to Y" means X or more and Y or less.

Although some embodiments of the invention have been described above, these embodiments are presented as examples and not intended to limit the scope of the invention. These embodiments can be implemented in other various modes, and, without departing from the gist of the invention, various omissions, substitutions, and changes can be made thereto. These embodiments, as well as omissions, substitutions, and changes thereto, etc., fall within the scope and gist of the invention, and also fall within the scope of the claimed invention and its equivalents.

## Claims

1. A gelling agent for gelling a polysaccharide, the gelling agent comprising a cellulose oligomer having an average degree of polymerization of 5 or more and 20 or less.

2. A gellable liquid composition comprising a cellulose oligomer having an average degree of polymerization of 5 or more and 20 or less, a polysaccharide, and water.

3. The liquid composition according to claim 2 gellable by neutralization with an acid, wherein the liquid composition is an alkaline aqueous solution containing the cellulose oligomer and the polysaccharide.

4. The liquid composition according to claim 2, obtained by neutralizing an alkaline aqueous solution containing the cellulose oligomer and the polysaccharide with an acid to precipitate the cellulose oligomer.

5. The liquid composition according to claim 2, wherein the polysaccharide is a non-cellulosic polysaccharide.

6. The liquid composition according to claim 2, wherein the polysaccharide is at least one selected from the group consisting of a glycosaminoglycan and a water-soluble polysaccharide that is composed only of glucose and/or a glucose derivative polymerized through an α-1,4-glycosidic bond and an α-1,6-glycosidic bond.

7. The liquid composition according to claim 2, comprising 0.5 to 100 parts by mass of the cellulose oligomer per 100 parts by mass of the polysaccharide.

8. A hydrogel formed by the gelation of the liquid composition according to any one of claims 2 to 7.
